(19) **Europäisches Patentamt / European Patent Office / Office européen des brevets**

(11) **EP 3 741 783 A1**

(12) **EUROPEAN PATENT APPLICATION**
published in accordance with Art. 153(4) EPC

(43) Date of publication:
**25.11.2020 Bulletin 2020/48**

(21) Application number: **19741630.8**

(22) Date of filing: **17.01.2019**

(51) Int Cl.:
**C08F 20/06** (2006.01)    **B01J 20/26** (2006.01)
**B01J 20/28** (2006.01)    **B01J 20/30** (2006.01)

(86) International application number:
**PCT/JP2019/001322**

(87) International publication number:
**WO 2019/142872 (25.07.2019 Gazette 2019/30)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.01.2018 JP 2018006396**

(71) Applicant: **Sumitomo Seika Chemicals Co., Ltd.**
**Kako-gun, Hyogo 675-0145 (JP)**

(72) Inventors:
• **CHIBA, Mikito**
  **Himeji-shi, Hyogo 672-8076 (JP)**
• **ONODA, Yuichi**
  **Himeji-shi, Hyogo 672-8076 (JP)**

(74) Representative: **Mewburn Ellis LLP**
**Aurora Building**
**Counterslip**
**Bristol BS1 6BX (GB)**

(54) **WATER-ABSORBENT RESIN**

(57)    The purpose of the present invention is to provide a water-absorbent resin having excellent absorption properties in a localized absorption area. Provided is a water-absorbent resin composed of a polymer of water-soluble ethylenically unsaturated monomers, wherein the physiological saline absorption rate is 5-30 seconds, and the diffusion absorption rate under load expressed as a physiological saline absorption capacity under a load of 1.96 kPa in a local area of 78.5 mm2 is at least 10 g/g. This water-absorbent resin has excellent absorption properties in a localized absorption area.

EP 3 741 783 A1

**Description**

TECHNICAL FIELD

The present invention relates to a water-absorbent resin. More specifically, the present invention relates to a water-absorbent resin to be suitably used for hygienic articles to be used for humans or animals, especially, light incontinence pads, vaginal discharge sheets, and urine absorbent articles for pets, because of its appropriate physiological saline absorption rate and physiological saline absorption capacity under load in a local area.

BACKGROUND ART

Water-absorbent resins are in use for materials (absorbent materials) in various fields, such as hygienic materials to be used for hygienic articles such as disposable diapers and feminine sanitary products, agricultural and horticultural materials to be used for water retention agents and soil conditioners, and industrial materials to be used for water stop agents and anti-moisture condensation agents.

As such a water-absorbent resin, a crosslinked product of a partially neutralized acrylic acid polymer has been suitably used. The crosslinked product of a partially neutralized acrylic acid polymer is a material that is not only superior in water-absorption capacity, but also in industrial aspects, such as that a resin having a fixed quality can be produced stably and inexpensively because of the easy industrial availability of acrylic acid, which is a raw material of the polymer, and that the resin is hardly degraded because of its high chemical stability.

Among the above-mentioned absorbent materials, hygienic materials to be used for hygienic articles such as disposable diapers and feminine sanitary products have therein an absorbent body mainly composed of hydrophilic fibers such as pulp and a water-absorbent resin. Due to the need to prevent liquid leakage for a relatively large amount of body fluids and the like, water-absorbent resins to be used for these hygienic articles have been improved to have better properties by a water absorption rate.

For the purpose of improving the water absorption rate of a water-absorbent resin, for example, Patent Document 1 discloses a method for producing a water-absorbent resin by polymerizing a water-soluble ethylenically unsaturated monomer that is a method of producing water-absorbent resin particles, characterized in that the polymerization is performed using a water-soluble azo type radical polymerization initiator in the presence of a polyvalent glycidyl compound as an internal-crosslinking agent, and then the resulting water-absorbent resin is post-crosslinked with a post-crosslinking agent. Patent Document 2 discloses, in order to solve a superior water absorption rate and also the problem of odor after water absorption, a method of producing water-absorbent resin particles having in order a first polymerization step including obtaining a hydrous gel polymer by polymerizing a water-soluble ethylenically unsaturated monomer in a suspension containing an oily liquid containing a hydrocarbon dispersion medium, a first aqueous liquid containing an aqueous solvent, the water-soluble ethylenically unsaturated monomer, and a radical polymerization initiator, and a surfactant having an HLB of 6 or more, in which the first aqueous liquid is dispersed in the oily liquid; and a second polymerization step including polymerizing a water-soluble ethylenically unsaturated monomer in a suspension prepared by mixing the suspension containing the hydrous gel polymer at a temperature of 45°C or more with a second aqueous liquid containing an aqueous solvent, the water-soluble ethylenically unsaturated monomer, and a radical polymerization initiator, thereby further dispersing the second aqueous liquid.

On the other hand, hygienic articles are diversifying according to the needs of the market; for example, many small hygienic articles such as light incontinence pads, vaginal discharge sheets, and urine absorbent articles for pets have been commercialized. The hygienic materials to be used for these small hygienic articles also have therein an absorbent body mainly composed of hydrophilic fibers such as pulp and a water-absorbent resin, and those used for disposable diapers and feminine sanitary products have been used. These small hygienic articles, especially, light incontinence pads and vaginal discharge sheets, are increasingly required to be thinner and lighter in terms of design, convenience in carrying, and distribution efficiency.

PRIOR ART DOCUMENTS

PATENT DOCUMENTS

Patent Document 1: JP-A-2006-176570
Patent Document 2: WO 2013/018571

## SUMMARY OF THE INVENTION

### PROBLEMS TO BE SOLVED BY THE INVENTION

Hygienic materials to be used for small hygienic articles such as light incontinence pads, vaginal discharge sheets, and urine absorbent articles for pets must have a small body fluid absorption area due to the design of the hygienic articles. That is, they are characteristic in that the absorption area is limited. In order to meet the needs for thinning and lightening of a hygienic material having such characteristics, it is necessary to have superior absorption properties in a limited absorption area. However, such absorption properties have not been studied with hygienic materials to be used for small hygienic articles.

For example, in the production method described in Patent Document 1, a high water absorption capacity under load and a water absorption rate of 45 to 50 seconds have actually been achieved by using a sucrose fatty acid ester having an HLB of 3.0 as a surfactant. However, when considering the application of such a water-absorbent resin to a small hygienic article, it is prone to leak liquid due to its excessively large absorption area, so that it does not satisfy water absorption properties.

In the production method described in Patent Document 2, a water absorption rate of 2 to 4 seconds has actually been achieved by using potassium persulfate as a polymerization initiator. However, when considering the application of such a water-absorbent resin to a small hygienic article, its water absorption capacity under load is small and it is prone to leak liquid due to its slow permeation rate in the second time (reabsorption), so that it does not satisfy absorption properties.

Thus, an object of the present invention is to provide a water-absorbent resin being superior in absorption property in a limited absorption area (hereinafter, sometimes referred to as local absorption property).

### MEANS FOR SOLVING THE PROBLEM

As a result of a diligent study, the present inventors found that the object of the present invention can be attained by a water-absorbent resin composed of a polymer of a water-soluble ethylenically unsaturated monomer, in which the physiological saline absorption rate and the physiological saline absorption capacity under load in a local area are within prescribed ranges. The present invention has been completed by repetitively conducting further studies on the basis of this finding.

In summary, the present invention provides aspects of invention as itemized below:

Item 1. A water-absorbent resin including a polymer of a water-soluble ethylenically unsaturated monomer, which has a physiological saline absorption rate of 5 to 30 seconds, and has a diffusing absorption rate under load, which is expressed as a physiological saline absorption capacity under a load of 1.96 kPa in a local area of 78.5 mm$^2$, of 10 g/g or more.

Item 2. The water-absorbent resin according to item 1, which has a physiological saline retention capacity of 35 g/g or more.

Item 3. The water-absorbent resin according to item 1 or 2, which has a median particle size of 200 to 800 $\mu$m.

Item 4. The water-absorbent resin according to any one of items 1 to 3, which has at least one shape selected from the group consisting of a granular shape, a substantially spherical shape, and a shape in which particles having a granular shape and/or a substantially spherical shape are aggregated.

Item 5. The water-absorbent resin according to any one of items 1 to 4, which is one obtained by a reversed phase suspension polymerization method.

Item 6. An absorbent article including an absorbent body including the water-absorbent resin according to any one of items 1 to 5 and a hydrophilic fiber.

### ADVANTAGES OF THE INVENTION

According to the present invention, there is provided a water-absorbent resin being superior in local absorption property due to being appropriate in physiological saline absorption rate and in physiological saline absorption capacity under load in a local area. Thus, even when applied to small hygienic articles such as light incontinence pads, vaginal discharge sheets, and urine absorbent articles for pets, high osmosis is achieved in a limited absorption area, so that diffusion of absorbed liquid is effectively suppressed and the risk of liquid leakage can be reduced.

### BRIEF DESCRIPTION OF THE DRAWINGS

Fig. 1 is a partially cutaway cross-sectional view schematically illustrating an apparatus for measuring a diffusing absorption rate under load of a water-absorbent resin.

Fig. 2 is a partially exploded view illustrating the configuration of the measuring apparatus depicted in Fig. 1.

EMBODIMENTS OF THE INVENTION

1.Water-absorbent resin

The water-absorbent resin of the present invention is a water-absorbent resin including a polymer of a water-soluble ethylenically unsaturated monomer, characterized by having a physiological saline absorption rate of 5 to 30 seconds and a diffusing absorption rate under load of 10 g/ g or more. The water-absorbent resin of the present invention will be hereinafter described in detail.

Physiological saline absorption rate

The water-absorbent resin of the present invention has a physiological saline absorption rate of 5 to 30 seconds. In both the cases where the physiological saline absorption rate is less than 5 seconds and more than 30 seconds, the local absorption properties deteriorate.

If the physiological saline absorption rate is excessively high, for example, a gel blocking phenomenon where a soft gel layer is formed near the surface of an absorbent body to prevent a liquid from penetrating into the interior of the absorbent body is likely to occur, so that the liquid stays in a surface layer portion of the absorbent body and liquid leakage easily occurs. Since the gel blocking phenomenon appears more remarkably under load, local absorption properties under load are particularly deteriorated. If the physiological saline absorption rate is excessively high, re-wetting, that is, easy leakage of the absorbed liquid out of the absorption surface of the absorbent body caused by receiving a load after absorption, is likely to occur. Since the re-wetting also appears more remarkably under load, the local absorption properties under load is particularly poor. From the viewpoint of more effectively suppressing such a phenomenon and obtaining better local absorption properties, the physiological saline absorption rate is 5 seconds or more, preferably 8 seconds or more, more preferably 10 seconds or more, and still more preferably 12 seconds or more.

If the physiological saline absorption rate is excessively low, for example, the absorption area is enlarged due to easy diffusion of liquid at the time of its absorption, so that liquid leakage from the end of an absorbent article is likely to occur. From the viewpoint of more effectively suppressing the diffusion of liquid during its absorption and obtaining better local absorption properties, the physiological saline absorption rate is 30 seconds or less, preferably 25 seconds or less, more preferably 20 seconds or less, still more preferably 19 seconds or less, even more preferably 16 seconds or less, and still even more preferably 14 seconds or less.

Specific ranges of the physiological saline absorption rate of the water-absorbent resin of the present invention include 5 to 25 seconds, 5 to 20 seconds, 5 to 19 seconds, 5 to 16 seconds, 5 to 14 seconds, 8 to 25 seconds, 8 to 20 seconds, 8 to 19 seconds, 8 to 16 seconds, 8 to 14 seconds, 10 to 25 seconds, 10 to 20 seconds, 10 to 19 seconds, 10 to 16 seconds, 10 to 14 seconds, 12 to 25 seconds, 12 to 20 seconds, 12 to 19 seconds, 12 to 16 seconds, and 12 to 14 seconds.

The physiological saline absorption rate referred to in the present invention is a value measured by the method described in the Examples described later.

Diffusing absorption rate under load

The water-absorbent resin of the present invention has a diffusing absorption rate under load of 10 g/g or more. The diffusing absorption rate under load referred to in the present invention is a value expressed as a physiological saline absorption capacity under a load of 1.96 kPa in a local area of 78.5 mm$^2$. More specifically, it is a value measured by the method described in the Examples described later.

When the diffusing absorption rate under load is less than 10 g/g, liquid stays in a surface layer of an absorbent body, so that problems are likely to occur, e.g., liquid leaks easily from an end of an absorbent article, re-wet easily occurs, or re-absorbing ability deteriorates, and local absorption properties deteriorate. From the viewpoint of obtaining better local absorption properties, the diffusing absorption rate under load is preferably 14 g/g or more, more preferably 15 g/g or more, and still more preferably 16 g/g or more. From the viewpoint of obtaining superior local absorption properties, the upper limit of the range of the diffusing absorption rate under load is, for example, 40 g/g or less, preferably 30 g/g or less, and more preferably 20 g/g or less.

Specific ranges of the diffusing absorption rate under load of the water-absorbent resin of the present invention include 10 to 40 g/g, 10 to 30 g/g, 10 to 20 g/g, 14 to 40 g/g, 14 to 30 g/g, 14 to 20 g/g, 15 to 40 g/g, 15 to 30 g/g, 15 to 20 g/g, 16 to 40 g/g, 16 to 30 g/g, and 16 to 20 g/g.

Physiological saline retention capacity

The water-absorbent resin of the present invention is particularly useful when the physiological saline retention capacity (g/g) is 35 g/g or more. Usually, an absorbent resin tends to have a reduced permeation rate and tends to be inferior in local absorption property if the water retention capacity of the resin is large, but the water-absorbent resin of the present invention can exhibit superior local absorption properties even when it has a water retention capacity as high as a physiological saline retention capacity of 35 g/g or more.

The physiological saline retention capacity (g/g) of the water-absorbent resin is preferably 36 g/g or more, and more preferably 38 g/g or more from the viewpoint of obtaining a higher retention capacity when used in an absorbent body. From the viewpoint of obtaining superior local absorption properties, the upper limit of the physiological saline retention capacity (g/g) is, for example, 70 g/g or less, preferably 60 g/g or less, more preferably 50 g/g or less, and still more preferably 45 g/g or less.

Specific ranges of the physiological saline retention capacity of the water-absorbent resin of the present invention include 36 to 70 g/g, 36 to 60 g/g, 36 to 50 g/g, 36 to 45 g/g, 38 to 70 g/g, 38 to 60 g/g, 38 to 50 g/g, and 38 to 45 g/g.

The physiological saline retention capacity referred to in the present invention is a value measured by the method described in the Examples described later.

Median particle size

From the viewpoint of obtaining superior local absorption properties when the water-absorbent resin of the present invention is used for an absorbent body, the lower limit of the median particle size of the water-absorbent resin is, for example, 200 $\mu$m or more, preferably 300 $\mu$m or more, more preferably 350 $\mu$m or more, still more preferably 400 $\mu$m or more, and even more preferably 420 $\mu$m or more, and the upper limit is, for example, 800 $\mu$m or less, preferably 600 $\mu$m or less, more preferably 500 $\mu$ or less, still more preferably 470 $\mu$m or less, and even more preferably 430 $\mu$m or less. The specific ranges of the physiological saline retention capacity of the water-absorbent resin of the present invention include 200 to 800 $\mu$m, 200 to 600 $\mu$m, 200 to 500 $\mu$m, 200 to 470 $\mu$m, 200 to 430 $\mu$m, 300 to 800 $\mu$m, 300 to 600 $\mu$m, 300 to 500 $\mu$m, 300 to 470 $\mu$m, 300 to 430 $\mu$m, 350 to 800 $\mu$m, 350 to 600$\mu$m, 350 to 500 $\mu$m, 350 to 470 $\mu$m, 350 to 430 $\mu$m, 400 to 800 $\mu$m, 400 to 600 $\mu$m, 400 to 500 $\mu$m, 400 to 470 $\mu$m, 400 to 430 $\mu$m, 420 to 800 $\mu$m, 420 to 600 $\mu$m, 420 to 500 $\mu$m, 420 to 470 $\mu$m, and 420 to 430 $\mu$m.

The median particle size referred to in the present invention is a value measured by the method described in the Examples described later.

Shape

Examples of the shape of the water-absorbent resin of the present invention include a granular shape, a substantially spherical shape, a shape in which resin having a granular shape or a substantially spherical shape is aggregated, a crushed indefinite shape, a shape in which resin having a crushed indefinite shape is aggregated, and a flat shape.

In the present invention, from the viewpoint of obtaining superior local absorption properties, the shape of the water-absorbent resin may be a particle shape. More specifically, it may be at least one shape selected from the group consisting of a granular shape, a spherical shape, a substantially spherical single particle shape such as an elliptical spherical shape; and shapes in which particles having a granular shape and/or a substantially spherical shape are aggregated. Water-absorbent resins having these shapes can be produced by a reversed phase suspension polymerization method or a spray droplet polymerization method. Among these shapes, a granular shape is preferable. Adjusting the hydrophile-lipophile balance (HLB) value of the surfactant to be used in the polymerization step preferably to 6 or more, more preferably to 7 or more, still more preferably to 8 or more, and even more preferably to 8.5 or more makes it easier to obtain a granular water-absorbent resin.

In addition to the above, the shape of the water-absorbent resin of the present invention also may be at least one shape selected from the group consisting of a crushed indefinite shape and a shape in which particles having a crushed indefinite shape are aggregated. The water-absorbent resin having such a shape can be produced, for example, by an aqueous solution polymerization method.

Local absorption property

Since the water-absorbent resin of the present invention has at least the above-described prescribed physiological saline absorption rate and the prescribed diffusing absorption rate under load, it is a water-absorbent resin being superior in absorption property in a limited absorption area, namely, local absorption property when applied to an absorbent article.

The local absorption property exhibited by the present invention may be that the diffusion area after the initial absorption and after re-absorption for an absorbent article are both small, the initial permeation rate to the absorbent article is good, the maintainability of the permeation rate after the re-absorption is good (the difference between the initial permeation rate and the permeation rate after re-absorption is small), and liquid re-wets a little after absorption into the absorbent article.

2. Absorbent article

The absorbent article of the present invention includes an absorbent body including the above-described water-absorbent resin and a hydrophilic fiber as an absorbent material.

Examples of the hydrophilic fiber include cellulose fibers such as cotton-like pulp and chemical pulp, and artificial cellulose fibers such as rayon and acetate. The absorbent body may further include synthetic fibers made of a synthetic resin such as polyamide, polyester, or polyolefin as a reinforcing agent. The structure of the absorbent body is not particularly limited, and examples thereof include a mixing structure in which a water-absorbent resin and hydrophilic fibers are uniformly blended, a sandwich structure in which a water-absorbent resin is held between a plurality of layers of hydrophilic fibers, and a structure in which a water-absorbent resin and hydrophilic fibers are wrapped in a water-permeable sheet such as a nonwoven fabric.

In the absorbent body, additives according to the purpose may be contained such that it is blended with the above-described water-absorbent resin. Examples of such additives include inorganic powders, metal chelating agents, antioxidants, antibacterial agents, and deodorants.

The absorbent article is preferably a hygienic article. The hygienic article is configured by including an absorbent body, and a liquid-permeable sheet and a liquid-impermeable sheet laminated such that the absorbent body is sandwiched therebetween.

The liquid-permeable sheet is disposed on the side where the absorbent body comes into contact with an object that discharges liquid, and the liquid-impermeable sheet is disposed opposite to the side where the absorbent body comes into contact with the object. Examples of the liquid-permeable sheet include a nonwoven fabric made of a synthetic resin such as polyethylene, polypropylene, polyester, and polyamide, and a porous synthetic resin sheet. Examples of the liquid-impermeable sheet include a sheet made of a synthetic resin such as polyethylene, polypropylene, and polyvinyl chloride, and a sheet made of a composite material of these synthetic resins and a nonwoven fabric.

Since the above-described water-absorbent resin is superior in local absorption property, specific example of a hygienic article include a relatively small hygienic article such as a light incontinence pad, a vaginal discharge sheet, and a urine absorbent article for pets. Among these, more preferred are smaller hygienic articles such as a light incontinence pad and a vaginal discharge sheet.

3. Method for producing water-absorbent resin

The water-absorbent resin of the present invention can be produced by polymerizing a water-soluble ethylenically unsaturated monomer.

Examples of the method of polymerizing the water-soluble ethylenically unsaturated monomer include an aqueous solution polymerization method, a spray droplet polymerization method, an emulsion polymerization method, and a reversed phase suspension polymerization method, which are typical polymerization methods. In the aqueous solution polymerization method, polymerization is carried out by heating an aqueous solution of a water-soluble ethylenically unsaturated monomer with stirring as necessary. In the reversed phase suspension polymerization method, polymerization is carried out by heating a water-soluble ethylenically unsaturated monomer with stirring in a hydrocarbon dispersion medium. Among these, from the viewpoint of more precisely controlling the physiological saline absorption rate and the diffusing absorption rate under load of the water-absorbent resin of the present invention, the aqueous solution polymerization method and the reversed phase suspension polymerization method are preferable, and the reverse phase suspension polymerization method is more preferable.

The reversed phase suspension polymerization method, which is a preferred example of the method for producing the water-absorbent resin of the present invention, will be described in more detail below.

The reversed phase suspension polymerization method includes a polymerization step of performing a polymerization reaction of a water-soluble ethylenically unsaturated monomer in the presence of a radical polymerization initiator in a hydrocarbon dispersion medium to obtain a hydrous gel, and a post-crosslinking step of post-crosslinking the hydrous gel in the presence of a post-crosslinking agent.

(Polymerization step)

In the polymerization step, a polymerization reaction of a water-soluble ethylenically unsaturated monomer is carried out in the presence of a radical polymerization initiator in a hydrocarbon dispersion medium to obtain a hydrous gel.

[Water-soluble ethylenically unsaturated monomer]

Examples of the water-soluble ethylenically unsaturated monomer include (meth)acrylic acid ("acryl" and "methacryl" are herein collectively referred to as "(meth)acryl"; the same applies below) and salts thereof; 2-(meth)acrylamido-2-methylpropanesulfonic acid and salts thereof; nonionic monomers such as (meth)acrylamide, N,N-dimethyl(meth)acrylamide, 2-hydroxyethyl(meth)acrylate, N-methylol(meth)acrylamide, and polyethylene glycol mono(meth)acrylate; and amino group-containing unsaturated monomers such as N,N-diethylaminoethyl(meth)acrylate, N,N-diethylaminopropyl(meth)acrylate, and diethylaminopropyl(meth)acrylamide, as well as quaternary compounds thereof. Preferred among these water-soluble ethylenically unsaturated monomers are (meth)acrylic acid and salts thereof, (meth)acrylamide, and N,N-dimethyl(meth)acrylamide, and more preferred are (meth)acrylic acid and salts thereof, from the viewpoint of being readily industrially available. These water-soluble ethylenically unsaturated monomers may be used alone or in combination of two or more.

Among these water-soluble ethylenically unsaturated monomers, acrylic acid and salts thereof are widely used as raw materials of water-absorbent resins. Copolymers of acrylic acid and/or salts thereof with other water-soluble ethylenically unsaturated monomers as mentioned above may also be used. In this case, an acrylic acid and/or a salt thereof as a main water-soluble ethylenically unsaturated monomer is preferably used in an amount of 70 to 100 mol% based on the total amount of all water-soluble ethylenically unsaturated monomers.

In the polymerization step, the water-soluble ethylenically unsaturated monomer is preferably prepared in the form of an aqueous solution and dispersed in a hydrocarbon dispersion medium. By using the water-soluble ethylenically unsaturated monomer in the form of an aqueous solution, the dispersion efficiency in the hydrocarbon dispersion medium can be increased. The concentration of the water-soluble ethylenically unsaturated monomer in the aqueous solution is preferably in the range of 20% by mass to not more than the saturation concentration. The concentration of the water-soluble ethylenically unsaturated monomer is more preferably 20% by mass to 55% by mass, still more preferably 28% by mass to 50% by mass, and even more preferably 30% by mass to 45% by mass.

When the water-soluble ethylenically unsaturated monomer has an acid group such as (meth)acrylic acid or 2-(meth)acrylamido-2-methylpropanesulfonic acid, the acid group may be neutralized with an alkaline neutralizing agent, as required, before use. Examples of such alkaline neutralizing agents include alkali metal salts such as sodium hydroxide, sodium carbonate, sodium hydrogen carbonate, potassium hydroxide, and potassium carbonate; and ammonia. These alkaline neutralizing agents may be used in the form of aqueous solutions to facilitate the neutralization operation. The above-mentioned alkaline neutralizing agents may be used alone or in combination of two or more.

The degree of neutralization of the water-soluble ethylenically unsaturated monomer with an alkaline neutralizing agent, calculated as the degree of neutralization of all acid groups in the water-soluble ethylenically unsaturated monomer, is preferably 10 to 100 mol%, more preferably 30 to 90 mol%, still more preferably 40 to 85 mol%, and even more preferably 50 to 80 mol%.

[Radical polymerization initiator]

Examples of the radical polymerization initiator to be used in the polymerization step include persulfates such as potassium persulfate, ammonium persulfate, and sodium persulfate; peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; and azo compounds such as 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, 2,2'-azobis{2-[1-(2-hydroxyethyl)-2-imidazolin-2-yl]propane} dihydrochloride, 2,2'-azobis{2-methyl-N-[1,1-bis(hydroxymethyl)-2-hydroxyethyl]propionamide}, 2,2'-azobis[2-methyl-N-(2-hydroxyethyl)-propionamide], 2,2'-azo-bis[N-(2-carboxyethyl)-2-methylpropionamidine] tetrahydrate, and 4,4'-azobis(4-cyanovaleric acid). Among these radical polymerization initiators, persulfates and azo compounds are preferably used from the viewpoint of being readily available and easy to handle, and further, from the viewpoint that the local absorption properties of a resulting water-absorbent resin are more preferably obtained, azo compounds are more preferably used. Among the azo compounds, 2,2'-azobis(2-amidinopropane) dihydrochloride, 2,2'-azobis{2-[1-(2-hydroxyethyl)-2-imidazolin-2-yl]propane} dihydrochloride, and 2,2'-azobis[N-(2-carboxyethyl)-2-methylpropionamidine] tetrahydrate are more preferably used.

The above-mentioned radical polymerization initiators may be used alone or in combination of two or more.

The above-mentioned radical polymerization initiators may also be used in combination with reducing agents such as sodium sulfite, sodium hydrogensulfite, ferrous sulfate, and L-ascorbic acid to be used as redox polymerization initiators.

The amount of the radical polymerization initiator to be used is, for example, 0.00005 to 0.01 mol, preferably 0.0001 to 0.01 mol, more preferably 0.0003 to 0.01 mol, per mole of the water-soluble ethylenically unsaturated monomer. The use of the radical polymerization initiator in the above-defined range of amounts can avoid the occurrence of an abrupt polymerization reaction, and can complete the polymerization reaction in an appropriate time.

[Internal-crosslinking agent]

In the polymerization step, a gel may be obtained in the form of a gel obtained by crosslinking a gel obtained in the polymerization step with an internal-crosslinking agent by placing an internal-crosslinking agent in the reaction solution as necessary.

Examples of the internal-crosslinking agent include those that can crosslink the polymer of the water-soluble ethylenically unsaturated monomer to be used, for example: unsaturated polyesters obtained by reacting polyols such as diols and triols, e.g., (poly)ethylene glycol ["(poly)" means both cases with and without the prefix "poly"; the same applies below], (poly)propylene glycol, 1,4-butanediol, trimethylolpropane, and (poly)glycerin, with unsaturated acids such as (meth)acrylic acid, maleic acid, and fumaric acid; bisacrylamides such as N,N-methylenebisacrylamide; di or tri(meth)acrylic acid esters obtained by reacting polyepoxides with (meth)acrylic acid; carbamyl di(meth)acrylates obtained by reacting polyisocyanates such as tolylene diisocyanate and hexamethylene diisocyanate with hydroxyethyl (meth)acrylate; compounds having two or more polymerizable unsaturated groups such as allylated starch, allylated cellulose, diallyl phthalate, N,N',N"-triallylisocyanurate, and divinylbenzene; polyglycidyl compounds such as diglycidyl compounds and triglycidyl compounds, e.g., (poly)ethylene glycol diglycidyl ether, (poly)propylene glycol diglycidyl ether, and (poly)glycerin diglycidyl ether; epihalohydrin compounds such as epichlorohydrin, epibromohydrin, and $\alpha$-methyl-epichlorohydrin; compounds having two or more reactive functional groups such as isocyanate compounds, e.g., 2,4-tolylene diisocyanate and hexamethylene diisocyanate; and oxetane compounds such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol. Among these internal-crosslinking agents, polyglycidyl compounds are preferably used, diglycidyl ether compounds are more preferably used, and (poly)ethylene glycol diglycidyl ether, (poly)propylene glycol diglycidyl ether, and (poly)glycerin diglycidyl ether are still more preferably used. These internal-crosslinking agents may be used alone or in combination of two or more.

The amount of the internal-crosslinking agent to be used is preferably 0.000001 to 0.02 mol, more preferably 0.000003 to 0.01 mol, still more preferably 0.000005 to 0.005 mol, even more preferably 0.000005 to 0.00001 mol, per mole of the water-soluble ethylenically unsaturated monomer.

[Hydrocarbon dispersion medium]

Examples of the hydrocarbon dispersion medium include aliphatic hydrocarbons having 6 to 8 carbon atoms such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. Among these hydrocarbon dispersion media, n-hexane, n-heptane, and cyclohexane are suitably used especially in terms of being readily industrially available, stable in quality, and inexpensive, and n-heptane is more suitably used.

These hydrocarbon dispersion media may be used alone or in combination of two or more. As examples of mixtures of hydrocarbon dispersion media, the use of commercially available products such as Exxsol heptane (produced by ExxonMobil Corporation; containing 75 to 85% by mass of heptane and its isomeric hydrocarbons) also leads to favorable results.

The amount of the hydrocarbon dispersion medium to be used is preferably 100 to 1500 parts by mass, and more preferably 200 to 1400 parts by mass per 100 parts by mass of the first-stage water-soluble ethylenically unsaturated monomer from the viewpoint of uniformly dispersing the water-soluble ethylenically unsaturated monomer, and facilitating control of the polymerization temperature. As described below, reversed phase suspension polymerization is carried out in a single stage or two or more stages. The first-stage polymerization as mentioned above refers to the first-stage polymerization reaction in single-stage polymerization or multi-stage polymerization (the same applies below).

[Dispersion stabilizer]

In the polymerization step, a dispersion stabilizer may be used to improve the dispersion stability of the water-soluble ethylenically unsaturated monomer in the hydrocarbon dispersion medium. Examples of the dispersion stabilizer include a surfactant and a polymeric dispersion agent.

Examples of usable surfactants include sucrose fatty acid esters, polyglycerin fatty acid esters, sorbitan fatty acid esters, polyoxyethylene sorbitan fatty acid esters, polyoxyethylene glycerin fatty acid esters, sorbitol fatty acid esters, polyoxyethylene sorbitol fatty acid esters, polyoxyethylene alkyl ethers, polyoxyethylene alkyl phenyl ethers, polyoxyethylene castor oil, polyoxyethylene hydrogenated castor oil, alkyl allyl formaldehyde condensate polyoxyethylene ethers, polyoxyethylene-polyoxypropylene block copolymers, polyoxyethylene polyoxypropyl alkyl ethers, polyethylene glycol fatty acid esters, alkyl glucosides, N-alkyl glyconamides, polyoxyethylene fatty acid amides, polyoxyethylene alkylamines, polyoxyethylene alkyl allyl ether phosphates, and polyoxyethylene alkyl phenyl ether phosphates. Among these surfactants, polyglycerin fatty acid esters, sorbitan fatty acid esters, polyoxyethylene alkyl ethers, and polyoxyethylene alkyl phenyl ether phosphates are particularly preferably used from the viewpoint of dispersion stability of the monomer. These surfactants may be used alone or in combination of two or more.

From the viewpoint of preferably obtaining the local absorption properties of a resulting water-absorbent resin, the hydrophile-lipophile balance (HLB) value of the surfactant is preferably 6 or more, more preferably 7 or more, still more preferably 8 or more, and even more preferably 8.5 or more. The upper limit of the HLB value is not particularly limited, but is preferably 16 or less, more preferably 12 or less. Specific ranges of HLB values include 6 to 16, 6 to 12, 7 to 16, 7 to 12, 8 to 16, 8 to 12, 8.5 to 16, and 8.5 to 12. The HLB value is commonly known as a value that indicates the affinity of a surfactant with water and oil, and can be determined by a known calculation method such as the Griffin method.

The amount of the surfactant to be used is preferably 0.1 to 30 parts by mass, more preferably 0.3 to 20 parts by mass per 100 parts by mass of the water-soluble ethylenically unsaturated monomer.

The polymeric dispersion agent may be used in combination with the surfactant described above. Examples of the polymeric dispersion agent include maleic anhydride-modified polyethylene, maleic anhydride-modified polypropylene, maleic anhydride-modified ethylene-propylene copolymers, maleic anhydride-modified EPDM (ethylene-propylene-diene terpolymers), maleic anhydride-modified polybutadiene, maleic anhydride-ethylene copolymers, maleic anhydride-propylene copolymers, maleic anhydride-ethylene-propylene copolymers, maleic anhydride-butadiene copolymers, polyethylene, polypropylene, ethylene-propylene copolymers, oxidized polyethylene, oxidized polypropylene, oxidized ethylene-propylene copolymers, ethylene-acrylic acid copolymers, ethyl cellulose, and ethyl hydroxyethyl cellulose. Among these polymeric dispersion agents, maleic anhydride-modified polyethylene, maleic anhydride-modified polypropylene, maleic anhydride-modified ethylene-propylene copolymers, maleic anhydride-ethylene copolymers, maleic anhydride-propylene copolymers, maleic anhydride-ethylene-propylene copolymers, polyethylene, polypropylene, ethylene-propylene copolymers, oxidized polyethylene, oxidized polypropylene, and oxidized ethylene-propylene copolymers are particularly preferably used, from the viewpoint of dispersion stability of the monomer. These polymeric dispersion agents may be used alone or in combination of two or more.

The amount of the polymeric dispersion agent to be used is preferably 0.1 to 30 parts by mass, and more preferably 0.3 to 20 parts by mass, per 100 parts by mass of the water-soluble ethylenically unsaturated monomer.

[Other components]

In the polymerization step, other components may be added, as desired, to the aqueous solution containing the water-soluble ethylenically unsaturated monomer. Examples of such other components include various additives such as thickeners, foaming agents, and chain transfer agents.

Examples of usable thickeners include hydroxyethylcellulose, hydroxypropylcellulose, methylcellulose, carboxymethylcellulose, polyacrylic acid, (partially) neutralized polyacrylic acid, polyethylene glycol, polyacrylamide, polyethyleneimine, dextrin, sodium alginate, polyvinyl alcohol, polyvinylpyrrolidone, and polyethylene oxide. By adjusting the viscosity of the aqueous solution by adding a thickener, it is possible to control the median particle size obtained in reverse phase suspension polymerization. For a fixed stirring rate during the polymerization, the higher the viscosity of the aqueous solution containing the water-soluble ethylenically unsaturated monomer, the larger the median particle size of the resulting particles tends to be.

Examples of usable foaming agents include carbonates such as calcium carbonate, magnesium carbonate, disodium carbonate, dipotassium carbonate, diammonium carbonate, and bicarbonates such as sodium bicarbonate and potassium bicarbonate. By adding a foaming agent to make the aqueous solution contain bubbles, it is possible to control the surface area of a resulting water-absorbent resin.

[Reaction operation]

A specific reaction operation in the polymerization step may be dispersing an aqueous monomer solution containing a water-soluble ethylenically unsaturated monomer in a hydrocarbon dispersion medium in the presence of a dispersion stabilizer. Examples of the method of dispersing the aqueous monomer solution containing the water-soluble ethylenically unsaturated monomer in the hydrocarbon dispersion medium include a method of adding the aqueous monomer solution containing the water-soluble ethylenically unsaturated monomer at once and a method of adding the aqueous monomer solution by dropping. When a dispersion stabilizer is used, the timing of adding the dispersion stabilizer to the hydrocarbon dispersion medium may be any time before the start of the polymerization reaction, and may be either of before and after the addition of the aqueous monomer solution to the hydrocarbon dispersion medium.

From the viewpoint of obtaining the local water absorption property of a resulting water-absorbent resin more preferably, a method of adding the aqueous monomer solution containing the water-soluble ethylenically unsaturated monomer by dropping is preferable.

In the case of adding the aqueous monomer solution containing the water-soluble ethylenically unsaturated monomer by dropping to the hydrocarbon dispersion medium, the dropping may be continuous or intermittent. Further, the dropping speed may be constant or may vary. From the viewpoint of making the polymerization reaction uniform and facilitating the control of the particle diameter, it is preferable that the dropping be continuous and at a constant rate.

Although the dropping time during which the aqueous monomer solution containing the water-soluble ethylenically unsaturated monomer is continuously added dropwise to the hydrocarbon dispersion medium varies depending on the type and the stirring rate of stirring blades described below or the synthesis scale, it is preferably 5 to 30 minutes, more preferably 5 to 20 minutes, still more preferably 6 to 15 minutes, and even more preferably 9 to 15 minutes. By adjusting the dropping time, a water-absorbent resin having a better local absorption property can be obtained. For example, the dropping time may be set as long as possible within the above-mentioned dropping time range in order to obtain a water-absorbent resin having a better local absorption property.

The polymerization step can be performed in a single stage or two or more multiple stages. In the case where the aqueous monomer solution is added dropwise, the individual stages are separated from each other by inserting other operations, such as cooling, drying, intermediate crosslinking, which can be performed by the same operations as those of the intermediate crosslinking step described below, or heat retention without dropwise addition, to between dropping operations and/or by varying the type of the monomer to be dropped. When the aqueous monomer solution is added at once, the individual stages such as the first stage and the second stage are distinguished for each addition of the monomer aqueous solution.

Reversed phase suspension polymerization with two or more multiple stages may be performed as follows: the first-stage reversed phase suspension polymerization is performed; subsequently, a water-soluble ethylenically unsaturated monomer is added to the reaction mixture obtained by the first-stage polymerization reaction and mixed, and reversed phase suspension polymerization in the second and subsequent stages is performed in the same manner as in the first stage. In reversed phase suspension polymerization in each of the second and subsequent stages, in addition to the water-soluble ethylenically unsaturated monomer, a radical polymerization initiator is preferably added within the above-described range of molar ratios of each of the components relative to the water-soluble ethylenically unsaturated monomer, based on the amount of the water-soluble ethylenically unsaturated monomer added during reversed phase suspension polymerization in each of the second and subsequent stages. In the second and subsequent stages of polymerization, an internal-crosslinking agent may also be added, as required, to the water-soluble ethylenically unsaturated monomer.

The reaction temperature during the polymerization step is preferably 20 to 100°C, and more preferably 40 to 90°C from the viewpoint of allowing the polymerization reaction to proceed quickly to reduce the polymerization time for improved economic efficiency, and readily removing the heat of polymerization to perform a smooth reaction.

The operation of stirring the aqueous monomer solution can be carried out using any of various well-known stirring blades. Specific examples of usable stirring blades include propeller blades, paddle blades, anchor blades, turbine blades, Pfaudler blades, ribbon blades, FULLZONE blades (manufactured by Shinko Pantec Co., Ltd.), MAXBLEND blades (manufactured by Sumitomo Heavy Industries, Ltd.), and SUPERMIX blades (manufactured by Satake Chemical Equipment Mfg., Ltd.). The median particle size of the particles obtained in the polymerization reaction can be controlled by adjusting the stirring rate, e.g., the rotation speed, in reversed phase suspension polymerization.

(Intermediate crosslinking step)

The reversed phase suspension polymerization method may further include an intermediate crosslinking step after the polymerization step and before the post-crosslinking step. In the intermediate crosslinking step, an intermediate crosslinking agent is mixed with the reaction mixture obtained in the polymerization reaction, and the mixture is heated to obtain a hydrous gel in an intermediately crosslinked state.

Examples of the intermediate crosslinking agent include the same ones as the above-described internal-crosslinking agent. The amount of the intermediate crosslinking agent to be used is preferably 0.00001 to 0.01 mol, more preferably 0.00002 to 0.005 mol, and still more preferably 0.0003 to 0.002 mol per mole of the total amount of the water-soluble ethylenically unsaturated monomer used for the polymerization.

As a method of adding the intermediate crosslinking agent, the intermediate crosslinking agent may be added as it is, may be added in the form of an aqueous solution, or may be added in the form of a solution prepared using water or a hydrophilic organic solvent. Examples of the hydrophilic organic solvent include lower alcohols such as methyl alcohol, ethyl alcohol, n-propyl alcohol, and isopropyl alcohol; ketones such as acetone and methyl ethyl ketone; ethers such as diethyl ether, dioxane, and tetrahydrofuran; amides such as N,N-dimethylformamide; and sulfoxides such as dimethyl sulfoxide. These hydrophilic organic solvents may be used alone or in combination of two or more.

(Post-crosslinking step)

In the post-crosslinking step, the hydrous gel obtained in the above-described step (specifically, the hydrogel obtained in the above-described polymerization step, or the hydrogel obtained in the above-mentioned intermediate crosslinking step and the intermediate crosslinked (Gel-like material) is post-crosslinked in the presence of a post-crosslinking agent. Thereby, a water-absorbent resin is obtained. By performing the post-crosslinking step, a water-absorbent resin can be achieved in which the crosslinking density in the vicinity of the surface has been increased to improve various kinds of performance such as the water absorption capacity under load.

Examples of the post-crosslinking agent include compounds having two or more reactive functional groups. Examples thereof include polyols such as ethylene glycol, propylene glycol, 1,4-butanediol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether (herein, (poly)ethylene glycol diglycidyl ether includes (poly)ethylene glycol diglycidyl ether and ethylene glycol diglycidyl ether; the same applies also to other compounds); haloepoxy compounds such as epichlorohydrin, epibromohydrin, and $\alpha$-methylepichlorohydrin; isocyanate compounds such as 2,4-tolylene diisocyanate and hexamethylene diisocyanate; oxetane compounds such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol; oxazoline compounds such as 1,2-ethylenebisoxazoline; carbonate compounds such as ethylene carbonate; and hydroxyalkylamide compounds such as bis[N,N-di($\beta$-hydroxyethyl)]adipamide. Among these post-crosslinking agents, polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether are preferred, (poly)ethylene glycol diglycidyl ether is more preferred, and ethylene glycol diglycidyl ether is still more preferred. These post-crosslinking agents may be used alone or in combination of two or more.

The amount of the post-crosslinking agent to be used is preferably 0.00001 to 0.01 mol, more preferably 0.00005 to 0.005 mol, and still more preferably 0.0001 to 0.002 mol per mole of the water-soluble ethylenically unsaturated monomer used in the polymerization. From the viewpoint of obtaining better local absorption properties, the amount of the post-crosslinking agent to be used is 0.0003 to 0.002 mol, and more preferably 0.0006 to 0.002 mol per mole of the total amount of the water-soluble ethylenically unsaturated monomer used in the polymerization.

As a method of adding the post-crosslinking agent, the post-crosslinking agent may be added either as it is or in the form of an aqueous solution, and as necessary, it may be added in the form of a solution prepared using a hydrophilic organic solvent as a solvent. Examples of the hydrophilic organic solvent include lower alcohols such as methyl alcohol, ethyl alcohol, n-propyl alcohol, and isopropyl alcohol; ketones such as acetone and methyl ethyl ketone; ethers such as diethyl ether, dioxane, and tetrahydrofuran; amides such as N,N-dimethylformamide; and sulfoxides such as dimethyl sulfoxide. These hydrophilic organic solvents may be used alone, in combination of two or more, or in the form of a mixed solvent with water.

As to the timing of the addition of the post-crosslinking agent, it is preferably added at any stage after the polymerization step of the water-soluble ethylenically unsaturated monomer in the presence of 1 to 400 parts by mass of water per 100 parts by mass of the water-soluble ethylenically unsaturated monomer, more preferably added in the presence of 5 to 200 parts by mass of water, still more preferably added in the presence of 10 to 100 parts by mass of water, and even more preferably added in the presence of 20 to 60 parts by mass of water. The amount of water herein refers to the total amount of the water contained in the reaction system and the water that is used, as required, during the addition of the post-crosslinking agent.

The reaction temperature during the post-crosslinking reaction is preferably 50 to 250°C, more preferably 60 to 180°C, still more preferably 60 to 140°C, and even more preferably 70 to 120°C. The reaction time of the post-crosslinking reaction is preferably 1 to 300 minutes, and more preferably 5 to 200 minutes.

(Drying step)

The water-absorbent resin obtained in the above-described post-crosslinking step is subjected to a drying step. In the drying step, a solvent such as water or a hydrocarbon dispersion medium is distilled off by externally applying energy such as heat. Specifically, by heating the system in which a hydrous gel is dispersed in a hydrocarbon dispersion medium, water and the hydrocarbon dispersion medium are once distilled off from the system by azeotropic distillation. At this time, returning only the removed hydrocarbon dispersion medium into the system makes continuous azeotropic distillation possible. This embodiment is preferable from the viewpoint that the resin is hardly degraded because the temperature in the system during drying is maintained at or below the azeotropic temperature with the hydrocarbon dispersion medium. Subsequently, water and the hydrocarbon dispersion medium are distilled off, and thus particles of a water-absorbent resin are obtained. By controlling the processing conditions in this drying step and thereby adjusting the amount of dehydration, it is possible to control various performances of a resulting water-absorbent resin.

In the drying step, the drying treatment may be performed either under normal pressure or under reduced pressure. From the viewpoint of increasing the drying efficiency, the drying treatment may be performed under a flow of gas such as nitrogen. When the drying treatment is performed under normal pressure, the drying temperature is preferably 70 to 250°C, more preferably 80 to 180°C, still more preferably 80 to 140°C, and even more preferably 90 to 130°C. In the case where the drying treatment is performed under reduced pressure, the drying temperature is preferably 40 to 160°C, and more preferably 50 to 110°C.

The drying step may be performed either after the post-crosslinking step or may simultaneously with the post-crosslinking step.

As necessary, various additives such as chelating agents, reducing agents, oxidizing agents, antibacterial agents, and deodorants may be added to the water-absorbent resin after the polymerization step, during the drying step or after the drying step.

EXAMPLES

The present invention will be hereinafter described in detail by way of examples and comparative examples. However, the present invention is not limited to these examples.

<Production of water-absorbent resin>

(Example 1)

A cylindrical round-bottomed separable flask having an inside diameter of 110 mm and equipped with a reflux condenser, a nitrogen gas inlet tube, and a stirrer having stirring blades (coated on their surface with a fluororesin) composed of two sets of four inclined paddle blades with a blade diameter of 50 mm was prepared. This flask was charged with 453 g of n-heptane as a hydrocarbon dispersion medium, and then 1.1 g of sorbitan monolaurate having an HLB of 8.6 (manufactured by NOF Corporation, Nonion LP-20R) as a surfactant was added thereto. The flask was immersed in a water bath at 80°C to heat with stirring. Subsequently, the atmosphere of the system was sufficiently replaced with nitrogen.

Separately, 92 g (1.03 mol) of an 80.5% by mass aqueous acrylic acid solution was placed in a 500-mL Erlenmeyer flask, and 147.7 g of a 20.9% by mass aqueous sodium hydroxide solution was added dropwise with external cooling to accomplish neutralization. Then, 0.10 g (0.00037 mol) of 2,2'-azobis(2-amidinopropane) dihydrochloride as an azo compound, and 0.0092 g (0.0000053 mol) of ethylene glycol diglycidyl ether as an internal-crosslinking agent were added and dissolved. As a result, an aqueous monomer solution was prepared.

Then, after sufficiently running nitrogen through the aqueous monomer solution, the rotation speed of the stirrer was adjusted to 1000 rpm, and the aqueous monomer solution was dropped at a fixed rate into the separable flask such that the dropping time of the whole aqueous monomer solution was 7 minutes. After completion of the dropping, 0.41 g (0.000047 mol) of a 2% by mass aqueous solution of ethylene glycol diglycidyl ether was added as an intermediate crosslinking agent, and the mixture was kept at 80°C for 20 minutes to afford a hydrous gel polymer.

Next, the reaction solution in the flask was heated in an oil bath at 125°C, and 95 g of water was distilled out of the system while refluxing n-heptane into the system by azeotropic distillation of n-heptane and water. Then, 4.14 g (0.00048 mol) of a 2% by mass aqueous solution of ethylene glycol diglycidyl ether was added as a post-crosslinking agent, and the mixture was kept at 80°C for 120 minutes. Subsequently, the mixture was dried by evaporating n-heptane to afford a water-absorbent resin. This resin was passed through a sieve with a mesh size of 850 $\mu$m to afford a granular water-absorbent resin. The median particle size of the resulting water-absorbent resin measured by the method described later was 445 $\mu$m.

(Example 2)

A granular water-absorbent resin was obtained in the same manner as in Example 1, except that the amount of the water distilled out of the system in the azeotropic distillation of n-heptane and water in Example 1 was changed to 102 g and the amount of the 2% by mass aqueous solution of ethylene glycol diglycidyl ether as a post-crosslinking agent was changed to 6.21 g (0.00071 mol). The median particle size of the resulting water-absorbent resin was 426 $\mu$m.

(Example 3)

A granular water-absorbent resin was obtained in the same manner as in Example 1, except that the dropping time of the whole amount of the aqueous monomer solution in Example 1 was changed to 10 minutes. The median particle size of the resulting water-absorbent resin was 468 $\mu$m.

(Comparative Example 1)

A cylindrical round-bottomed separable flask having an inside diameter of 110 mm, and equipped with a reflux condenser, a nitrogen gas inlet tube, and a stirrer having stirring blades composed of two sets of four inclined paddle blades with a blade diameter of 50 mm was prepared. This flask was charged with 300 g of n-heptane as a hydrocarbon dispersion medium, followed by addition of 0.74 g of a sucrose stearate having an HLB of 3 (RYOTO Sugar Ester S-370 available from Mitsubishi-Kagaku Foods Corporation) as a surfactant and 0.74 g of a maleic anhydride-modified ethylene-propylene copolymer (Hi-wax 1105A available from Mitsui Chemicals, Inc.) as a polymeric dispersion agent, and then the temperature was raised to 80°C with stirring to dissolve the surfactant, and then cooled to 50°C.

Separately, 92 g (1.02 mol) of an 80% by mass aqueous acrylic acid solution was placed in a 500-mL Erlenmeyer flask, and 102.2 g of a 30% by mass aqueous sodium hydroxide solution was added dropwise with external cooling to accomplish neutralization. Then, 0.092 g of hydroxyethylcellulose (HEC AW-15F available from Sumitomo Seika Chemicals Co. Ltd.) as a thickener, 0.074 g (0.000274 mol) of potassium persulfate as a peroxide, 0.010 g (0.000058 mol) of ethylene glycol diglycidyl ether as an internal-crosslinking agent, and 43.8 g of ion-exchanged water were added and dissolved. As a result, a first-stage aqueous monomer solution was prepared.

Subsequently, the rotation speed of the stirrer was adjusted to 500 rpm. Then, the first-stage aqueous monomer solution prepared as described above was added into the separable flask, and the atmosphere within the system was sufficiently replaced with nitrogen. The flask was subsequently immersed in a water bath at 70°C and heated and polymerization was performed for 60 minutes. As a result, a first-stage hydrous gel polymer was obtained.

Separately, 128.8 g (1.43 mol) of an 80% by mass aqueous acrylic acid solution was placed in another 500-mL Erlenmeyer flask, and 143.1 g of a 30% by mass aqueous sodium hydroxide solution was added dropwise with external cooling to accomplish neutralization. Then, 0.104 g (0.000382 mol) of potassium persulfate as a peroxide, 0.012 g (0.000067 mol) of ethylene glycol diglycidyl ether as an internal-crosslinking agent, and 15.9 g were added and dissolved. As a result, a second-stage aqueous monomer solution was prepared.

The rotation speed of the stirrer was changed to 1000 rpm, and then the atmosphere within the separable flask was cooled to 25°C. The entire amount of the second-stage aqueous monomer solution was added to the first-stage polymerization slurry at once, and the atmosphere within the system was sufficiently replaced with nitrogen. The flask was again immersed in a water bath at 70°C and heated, and the second-stage polymerization was performed for 30 minutes.

After the second-stage polymerization, the reaction solution was heated in an oil bath at 125°C, and 257 g of water was distilled out of the system while refluxing n-heptane by azeotropic distillation of n-heptane and water. Then, 4.42 g (0.00051 mol) of a 2% by mass aqueous solution of ethylene glycol diglycidyl ether was added as a post-crosslinking agent, and the mixture was kept at 80°C for 120 minutes. Subsequently, the mixture was dried by evaporating n-heptane to afford a water-absorbent resin. This resin was passed through a sieve with a mesh size of 850 $\mu$m to afford a water-absorbent resin in which spherical particles were agglomerated. The median particle size of the resulting water-absorbent resin was 368 $\mu$m.

(Comparative Example 2)

A cylindrical round-bottomed separable flask having an inside diameter of 110 mm and equipped with a reflux condenser, a nitrogen gas inlet tube, and a stirrer having stirring blades (coated on their surface with a fluororesin) composed of two sets of four inclined paddle blades with a blade diameter of 50 mm was prepared. This flask was charged with 400 g of n-heptane as a hydrocarbon dispersion medium, and then 0.83 g of sorbitan monolaurate (manufactured by NOF Corporation, tradename: Nonion LP-20R; HLB: 8.6) as a surfactant was added thereto. The flask was heated to 45°C with stirring and the surfactant was dissolved in n-heptane.

Separately, 81.0 g (0.91 mol) of an 80.5% by mass aqueous acrylic acid solution as a water-soluble ethylenically unsaturated monomer was placed in a 500-mL Erlenmeyer flask. While the aqueous acrylic acid solution was externally cooled with ice water, 130.0 g of a 20.9% by mass aqueous sodium hydroxide solution was added dropwise to perform neutralization. Then, 0.09 g (0.00033 mol) of potassium persulfate as a radical polymerization initiator was added and dissolved in a beaker. As a result, a first-stage aqueous monomer solution was prepared.

Subsequently, the rotation speed of the stirrer was adjusted to 450 rpm. Then, the entire amount of the first-stage aqueous monomer solution was added into the round-bottomed flask, and the atmosphere within the system was sufficiently replaced with nitrogen. The separable flask was subsequently immersed in a water bath at 70°C and the temperature in the system was raised and a polymerization reaction was performed for one hour. As a result, a first-stage hydrous gel polymer was obtained.

Separately, 97.2 g (1.35 mol) of an 80.5% by mass aqueous acrylic acid solution as a water-soluble ethylenically unsaturated monomer was added to another 500-mL Erlenmeyer flask. While the aqueous acrylic acid solution was cooled with ice water, 149.4 g of a 27.1% by mass aqueous sodium hydroxide solution was added dropwise to perform neutralization. Then, 0.11 g (0.00041 mol) of potassium persulfate as a water-soluble radical polymerization initiator was added and dissolved in a beaker. As a result, a second-stage aqueous monomer solution was prepared.

The suspension after the completion of the crosslinking reaction with an intermediate crosslinking agent was cooled to 70°C with stirring at a rotation speed of the stirrer of 700 rpm. The entire amount of the second-stage aqueous monomer solution was added to the cooled round-bottomed flask at once, and then the atmosphere within the system was sufficiently replaced with nitrogen gas while the temperature within the system was maintained at the temperature at which the addition was completed (55°C). The round-bottomed flask was immersed in a water bath at 70°C to raise the temperature within the system, and then polymerization was performed for one hour. As a result, a hydrous gel polymer was obtained.

Next, the flask was heated in an oil bath at 120°C, and 175.6 g of water was distilled out of the system while refluxing n-heptane into the system by azeotropic distillation of water and n-heptane. Then, 7.29 g (0.00084 mol) of a 2% by mass aqueous ethylene glycol diglycidyl ether solution as a post-crosslinking agent was added to the flask, and the mixture was kept at 80°C for 120 minutes. Subsequently, the mixture was dried by evaporating n-heptane to afford a water-absorbent resin powder. This resin powder was passed through a sieve with a mesh size of 850 $\mu$m to afford a granular water-absorbent resin. The median particle size of the resulting water-absorbent resin was 312 $\mu$m.

(Comparative Example 3)

A cylindrical round-bottomed separable flask having an inside diameter of 110 mm and equipped with a reflux condenser, a nitrogen gas inlet tube, and a stirrer having stirring blades (coated on their surface with a fluororesin) composed of two sets of four inclined paddle blades with a blade diameter of 50 mm was prepared. This flask was charged with 453 g of n-heptane as a hydrocarbon dispersion medium, and then 1.1 g of sucrose fatty acid ester having an HLB of 9 (manufactured by Mitsubishi-Kagaku Foods Corporation, RYOTO Sugar Ester S-970) as a surfactant was added thereto. The flask was immersed in a water bath at 80°C to heat with stirring. Subsequently, the atmosphere of the system was sufficiently replaced with nitrogen.

Separately, 92 g (1.03 mol) of an 80.5% by mass aqueous acrylic acid solution as a water-soluble ethylenically unsaturated monomer was placed in a 500-mL Erlenmeyer flask. While the aqueous acrylic acid solution was externally cooled, 147.7 g of a 20.9% by mass aqueous sodium hydroxide solution was added dropwise to perform neutralization. Then, 0.10 g (0.00037 mol) of potassium persulfate as a radical polymerization initiator and 0.0092 g (0.0000053 mol) of ethylene glycol diglycidyl ether as an internal-crosslinking agent were added and dissolved in a beaker. As a result, an aqueous monomer solution was prepared.

Then, after sufficiently running nitrogen through the aqueous monomer solution, the rotation speed of the stirrer was adjusted to 1000 rpm, and the aqueous monomer solution was dropped at a fixed rate into the separable flask such that the dropping time of the whole aqueous monomer solution was 7 minutes. After the completion of the dropwise addition, the polymer was immersed in a water bath at 80°C and kept warm for 20 minutes and thus a hydrous gel polymer was obtained.

Next, the reaction solution in the flask was heated in an oil bath at 125°C, and 95 g of water was distilled out of the system while refluxing n-heptane into the system by azeotropic distillation of n-heptane and water. Then, 4.14 g (0.00048 mol) of a 2% by mass aqueous solution of ethylene glycol diglycidyl ether was added as a post-crosslinking agent, and the mixture was kept at 80°C for 120 minutes. Subsequently, the mixture was dried by evaporating n-heptane to afford a water-absorbent resin powder. This resin powder was passed through a sieve with a mesh size of 850 $\mu$m to afford a granular water-absorbent resin. The median particle size of the resulting water-absorbent resin was 368 $\mu$m.

(Comparative Example 4)

A 2-L cylindrical round-bottomed separable flask having an inside diameter of 110 mm and equipped with a reflux condenser, a nitrogen gas inlet tube, and a stirrer having stirring blades composed of two sets of four inclined paddle blades with a blade diameter of 50 mm was prepared. This flask was charged with 300 g of n-heptane as a hydrocarbon dispersion medium, followed by addition of 0.74 g of a sucrose stearate having an HLB of 3 (RYOTO Sugar Ester S-370 available from Mitsubishi-Kagaku Foods Corporation) as a surfactant and 0.74 g of a maleic anhydride-modified ethylene-propylene copolymer (Hi-wax 1105A available from Mitsui Chemicals, Inc.) as a polymeric dispersion agent, and then the temperature was raised to 80°C with stirring to dissolve the surfactant, and then cooled to 50°C.

Separately, 92 g (1.02 mol) of an 80% by mass aqueous acrylic acid solution was placed in a 500-mL Erlenmeyer flask, and 146.0 g of a 21% by mass aqueous sodium hydroxide solution was added dropwise with external cooling to accomplish neutralization. Then, 0.092 g of hydroxyethylcellulose (HEC AW-15F available from Sumitomo Seika Chemicals Co. Ltd.) as a thickener, 0.11 g (0.00041 mol) of 2,2'-azobis(2-amidinopropane) dihydrochloride as an azo-based compound, and 0.0064 g (0.000037 mol) of ethylene glycol diglycidyl ether as an internal-crosslinking agent were added and dissolved. As a result, a first-stage aqueous monomer solution was prepared.

Subsequently, the rotation speed of the stirrer was adjusted to 500 rpm. Then, the first-stage aqueous monomer solution prepared as described above was added into the separable flask, and the atmosphere within the system was sufficiently replaced with nitrogen. The flask was subsequently immersed in a water bath at 70°C and heated, so that polymerization was started. Next, at the time when the temperature within the system had reached a peak temperature (80 to 90°C) of polymerization, the rotation speed of the stirrer was changed to 1000 rpm, and 92 g of water was distilled out of the system in an oil bath at 125°C while refluxing n-heptane into the system by azeotropic distillation of water and n-heptane. As a result, a first-stage hydrous gel polymer was obtained.

Separately, 128.8 g (1.43 mol) of an 80% by mass aqueous acrylic acid solution was placed in another 500-mL Erlenmeyer flask, and 159.0 g of a 27% by mass aqueous sodium hydroxide solution was added dropwise with external cooling to accomplish neutralization. Then, 0.11 g (0.00041 mol) of 2,2'-azobis(2-amidinopropane) dihydrochloride as an azo-based compound, and 0.0116 g (0.000067 mol) of ethylene glycol diglycidyl ether as an internal-crosslinking agent were added and dissolved. As a result, a second-stage aqueous monomer solution was prepared.

The rotation speed of the stirrer was changed to 1000 rpm, and then the atmosphere within the separable flask was cooled. The entire amount of the second-stage aqueous monomer solution was added to the first-stage polymerization slurry at once, and the atmosphere within the system adjusted to 27°C was sufficiently replaced with nitrogen. The flask was again immersed in a water bath at 70°C and heated, and the second-stage polymerization was performed for 30 minutes.

After the second-stage polymerization, the reaction solution in the flask was heated in an oil bath at 125°C, and 168 g of water was distilled out of the system while refluxing n-heptane into the system by azeotropic distillation of n-heptane and water. Then, 4.42 g (0.00051 mol) of a 2% by mass aqueous solution of ethylene glycol diglycidyl ether was added as a post-crosslinking agent, and the mixture was kept at 80°C for 120 minutes. Subsequently, the mixture was dried by evaporating n-heptane to afford a water-absorbent resin. This resin was passed through a sieve with a mesh size of 850 $\mu$m to afford a water-absorbent resin in which spherical particles were aggregated. The median particle size of the resulting water-absorbent resin was 370 $\mu$m.

<Measurement of water-absorbent resin>

The obtained water-absorbent resin was measured for physiological saline absorption rate, diffusing absorption rate under load, physiological saline retention capacity, median particle size, and absorption rate under pressure by the following methods.

(Physiological saline absorption rate)

The measurement of the physiological saline absorption rate was performed in a room adjusted to 25°C ± 1°C. Particles of a water-absorbent resin are classified in advance with JIS standard sieves. The water-absorbent resin on a sieve with a mesh size of 250 $\mu$m that pass through a sieve with a mesh size of 500 $\mu$m are used as resin samples. Next, in a 100 mL beaker, 50 ± 0.1 g of physiological saline adjusted to a temperature of 25 ± 0.2°C in a thermostatic water bath was stirred with a magnetic stirrer bar (without a ring of 8 mm in diameter, 30 mm in length) at a rotation speed of 600r/min, so that a vortex was generated. While stirring as described above, 2.0 ± 0.002 g of the resin sample was added to the physiological saline at once, and the time (seconds) taken until the vortex disappeared and the liquid surface was flattened since the addition of the resin sample was measured, and the time was defined as a physiological saline absorption rate.

(Diffusing absorption rate under load)

The measurement of the diffusing absorption rate under load was carried out in a room adjusted to 25°C ± 1°C using a measuring apparatus shown in Figs 1 and 2. Particles of a water-absorbent resin are classified in advance with JIS standard sieves. The water-absorbent resin on a sieve with a mesh size of 250 $\mu$m that pass through a sieve with a mesh size of 500 $\mu$m are used as resin samples. Next, a glass filter 7 having an outer diameter of 90 mm and a thickness of 5 mm was placed on a petri dish 6 having an inner diameter of 150 mm and a height of 30 mm, and physiological saline was poured to the height of the glass filter 7. A filter paper 8 (ADVANTEC No. 2) and a plastic (PET) sheet 9 with an outer diameter of 80 mm and a thickness of 0.1 mm having a circular hole H with a diameter of 10 mm (opening area: 78.5 mm$^2$) at the center were put on the glass filter 7.

Separately, 2.0 $\pm$ 0.002 g of a resin sample (water-absorbent resin) 5 was placed in a plastic cylinder 91 with an inner diameter of 60 mm and a height of 70 mm in which a 400-mesh stainless steel wire mesh 93 was attached to the bottom, and was leveled to be uniform in thickness on the bottom (wire mesh 93). Then, a piston 92 was inserted, a weight 90 was placed on the piston 92, and thus a load of 20 g/cm$^2$ (1.96 kPa) was uniformly applied to the resin sample (water-absorbent resin) 5. In this state, the mass (Wa) was measured. That is, the mass Wa is the total mass of the weight 90, the cylinder 91, the piston 92, the wire mesh 93, and the resin sample (water-absorbent resin) 5. The cylinder 91 with a load applied by the weight 90 was placed on the PET sheet 9, and physiological saline was absorbed for 60 minutes, and then the mass (Wb) was measured. That is, the mass Wb is the total mass of the weight 90, the cylinder 91, the piston 92, the wire mesh 93, the resin sample (water-absorbent resin) 5, and the physiological saline absorbed in the resin sample (water-absorbent resin) 5. The diffusing absorption rate under load was calculated from the following equation.

[Equation 1]

$$\text{Diffusing absorption rate under load (g/g)} = (Wb - Wa)/\text{Mass of water-absorbent resin}$$

(Physiological saline retention capacity)

The physiological saline retention capacity was measured in a room adjusted to 25°C $\pm$ 1°C. A 500-ml beaker was charged with 500 g of a 0.9% by mass aqueous sodium chloride solution (physiological saline) and 2.0 $\pm$ 0.002 g of the water-absorbent resin was dispersed therein with stirring using a magnetic stirrer bar (8 mm in diameter, 30 mm in length, without a ring) at a rotation speed of 600 rpm such that no unswollen lumps were formed. The dispersion was allowed to stand with stirring for 30 minutes, so that the water-absorbent resin was sufficiently swollen. The dispersion was subsequently poured into a cotton bag (Cottonbroad No. 60, 100 mm in width, 200 mm in length), and the top of the cotton bag was closed with a rubber band. Then, the cotton bag was dehydrated for one minute using a dehydrator (product number: H-122, manufactured by KOKUSAN Co., Ltd.) set at a centrifugal force of 167 G, and the mass Wc (g) of the dehydrated cotton bag containing the swollen gel was measured. The same operation was performed without adding the water-absorbent resin, and the mass Wd (g) of the empty cotton bag upon wetting (namely, the mass of a wet cotton bag obtained by dehydration after pouring physiological saline into an empty cotton bag) was measured. The physiological saline retention capacity of the water-absorbent resin was calculated from the following equation.

[Equation 2]

$$\text{Physiological saline retention capacity (g/g)} = [Wc - Wd]/\text{Mass of water-absorbent resin (g)}$$

(Median particle size)

JIS standard sieves having mesh sizes of 850 $\mu$m, 500 $\mu$m, 300 $\mu$m, 250 $\mu$m, 212 $\mu$m, 106 $\mu$m, and 75 $\mu$m, and a receiving tray were combined in that order from the top.

50 g of the water-absorbent resin was placed on the top sieve of the combined sieves, and shaken for 20 minutes with a Ro-Tap shaker to conduct classification. After the classification, the particle size distribution was determined by calculating the mass of the water-absorbent resin remaining on each of the sieves as the mass percentage relative to the total mass. With regard to this particle size distribution, the mass percentage of the water-absorbent resin remaining on each of the sieves was integrated in descending order of particle diameter. Thereby, the relationship between the sieve mesh size and the integrated value of the mass percentage of the water-absorbent resin remaining on each of the sieves was plotted on logarithmic probability paper. The plots on the probability paper were connected by straight lines, and a particle diameter equivalent to 50% by mass of the integrated mass percentage was determined as the median particle size.

(Absorption rate under pressure)

The measurement of the absorption rate under pressure was performed in a room adjusted to 25°C ± 1°C. A glass filter having an outer diameter of 90 mm and a thickness of 5 mm was placed on a petri dish having an inner diameter of 150 mm and a height of 30 mm, and physiological saline was poured to the height of the glass filter, and a filter paper (No. 2 produced by ADVANTEC) was placed thereon.

Separately, 0.9 ± 0.002 g of water-absorbent resin was uniformly scattered on the bottom of a plastic cylinder with an inner diameter of 60 mm and a height of 70 mm in which a 400-mesh stainless steel wire mesh was attached to the bottom, and a piston and a weight were placed thereon to add a load of 21 g/cm$^2$ (2.06 kPa) uniformly to the water-absorbent resin, and the mass (We) was measured. That is, the mass We is the total mass of the weight, the cylinder, the piston, the wire mesh, and the water-absorbent resin. The cylinder was placed on the filter paper, and physiological saline was absorbed for 60 minutes, and its mass (Wf) was measured. That is, the mass Wf is the total mass of the weight, the cylinder, the piston, the wire mesh, the water-absorbent resin, and the physiological saline absorbed in the water-absorbent resin. The absorption rate under pressure (2.06 kPa) was calculated from the following equation.

[Equation 3]

$$\text{Absorption rate under pressure (g/g)} = (\text{Wf} - \text{We})/\text{Mass of water-absorbent resin}$$

In addition, the same operation was performed as described above while changing the load applied to the water-absorbent resin from 21 g/cm$^2$ (2.06 kPa) to 49 g/cm$^2$ (4.81 kPa), and the absorption rate under pressure (4.81 kPa) was calculated.

<Preparation of absorbent article>

The preparation of an absorbent article was performed in a room adjusted to 25°C ± 1°C and a relative humidity RH of 50% or less. First, an absorbent body core in the shape of a sheet of 40 cm × 20 cm was produced using 2.4g of each water-absorbent resin obtained in Examples 1 to 3 and Comparative Examples 1 to 4 and 9.6g of crushed pulp (Rayfloc produced by Rayonier Inc.). Next, while the absorbent body core was placed between two tissue papers, each of which had the same size as the absorbent body core and a basis weight of 16 g/m$^2$, the absorbent body core was all over pressed with a load of 196 kPa for 30 seconds, and thus an absorbent body was prepared. In addition, a polyethylene-polypropylene air-through type porous liquid-permeable sheet having the same size as the absorbent body and a basis weight of 22g/m$^2$ was disposed on the upper surface of the absorbent body, and a polyethylene liquid-impermeable sheet having the same size and the same basis weight as the polyethylene-polypropylene air-through type porous liquid-permeable sheet was disposed on the lower surface of the absorbent body, and an absorbent article was formed by sandwiching the absorbent body therebetween.

<Evaluation of absorbent article>

The local absorption property of the prepared absorbent articles was evaluated by the following measurements of total permeation rate, amount of re-wet, and total diffusion area. The measurements of the total permeation rate, the amount of re-wet, and the total diffusion area of the absorbent article were performed in a room adjusted to 25°C ± 1°C and a relative humidity RH of 50% or less.

(Total permeation rate)

First, an absorbent article was placed on a horizontal table. A measurement device (60 g in mass) equipped with a liquid introduction cylinder having an inner diameter of 3 cm was placed on a central part of the absorbent article, and 20 ml of physiological saline colored with a small amount of Blue No. 1 was introduced into the cylinder in 2 seconds. A time taken until when the physiological saline completely disappeared in the cylinder was measured with a stopwatch, and it was defined as a first (initial) permeation rate (in second). Subsequently, the cylinder was removed and the absorbent article was left as it was on the table. After 180 seconds from the commencement of first physiological saline introduction, the same operation as in the first time was carried out with the measurement device at the same position and a second (reabsorption) permeation rate (in second) was measured. The sum total of the first (initial) and second (reabsorption) permeation times was taken as the total permeation rate. The figures after the decimal point were rounded off.

(Amount of re-wet)

The operations to the second physiological saline injection in the above-described method for measuring the total permeation rate were performed in the same manner, and at a time of 180 seconds after the commencement of the second test liquid injection, a filter paper bundle with a size of 10 cm × 10 cm, whose mass Wg (g), specifically being about 38 g, had been measured in advance, was placed on the absorbent article at the position where the physiological saline was introduced. Then, a weight with a mass of 1 kg having a 10 cm × 10 cm bottom surface was placed thereon. After the load was applied for 30 seconds, the mass of the filter paper bundle (Wh (g), that is, the total mass of the filter paper bundle of Wg (g) and the water absorbed by it) was measured, and the increased mass was defined as the amount of re-wet (g). The figures after the decimal point were rounded off.

(Total diffusion area)

After the first (initial) permeation rate measurement in the total permeation rate measurement described above, the maximum spread dimensions (cm) in the longitudinal direction and the latitudinal direction of the area of the absorbent article surface where the physiological saline had permeated (that is, the area colored through the permeation of the physiological saline) were measured, and the product of the maximum spread dimensions (cm) in the longitudinal direction and the latitudinal direction was defined as a diffusion area (initial). In addition, after the above-described measurement of the amount of re-wet, the maximum spread dimensions (cm) in the longitudinal direction and the latitudinal direction of the area of the absorbent article surface where the physiological saline had permeated (that is, the area colored through the permeation of the physiological saline) were measured, and the product of the maximum spread dimensions (cm) in the longitudinal direction and the latitudinal direction was defined as a diffusion area (reabsorption). Further, the sum total of the diffusion area (initial) and the diffusion area (reabsorption) was defined as a total diffusion area (cm$^2$). The figures after the decimal point were rounded off.

The summary of the water-absorbent resins produced in Examples and Comparative Examples and the results measured by the above-described measurement methods are shown in Table 1, and the results obtained by evaluating the absorbent articles prepared from the water-absorbent resins by the above-described evaluation methods are shown in Table 2.

[Table 1]

| | Physiological saline absorption rate (sec) | Diffusing absorption rate under load (g/g) | Physiological saline retention capacity (g/g) | Median particle size | Absorption rate under pressure (2.06 kPa) (g/g) | Absorption rate under pressure (4.81 kPa) (g/g) |
|---|---|---|---|---|---|---|
| Example 1 | 13 | 11 | 46 | 445 | 35 | 14 |
| Example 2 | 13 | 16 | 39 | 426 | 36 | 18 |
| Example 3 | 18 | 15 | 41 | 468 | 33 | 16 |
| Comparative Example 1 | 36 | 13 | 40 | 368 | 34 | 20 |
| Comparative Example 2 | 8 | 6 | 39 | 312 | 25 | 11 |
| Comparative Example 3 | 16 | 8 | 40 | 368 | 17 | 8 |
| Comparative Example 4 | 46 | 18 | 46 | 370 | 30 | 22 |

[Table 2]

| | Permeation rate (initial) (sec) | Permeation rate (re-absorption) (sec) | Total permeation rate (sec) | Re-wet (g) | Diffusion area (initial) (cm$^2$) | Diffusion area (re-absorption) (cm$^2$) | Total diffusion area (cm$^2$) |
|---|---|---|---|---|---|---|---|
| Example 1 | 11 | 18 | 29 | 6 | 232 | 382 | 614 |

(continued)

| | Permeation rate (initial) (sec) | Permeation rate (re-absorption) (sec) | Total permeation rate (sec) | Re-wet (g) | Diffusion area (initial) (cm$^2$) | Diffusion area (re-absorption) (cm$^2$) | Total diffusion area (cm$^2$) |
|---|---|---|---|---|---|---|---|
| Example 2 | 10 | 17 | 27 | 5 | 210 | 376 | 586 |
| Example 3 | 11 | 17 | 28 | 5 | 220 | 388 | 608 |
| Comparative Example 1 | 12 | 25 | 37 | 1 | 289 | 402 | 691 |
| Comparative Example 2 | 12 | 24 | 36 | 11 | 214 | 399 | 624 |
| Comparative Example 3 | 11 | 28 | 39 | 1 | 269 | 408 | 677 |
| Comparative Example 4 | 13 | 32 | 45 | 1 | 297 | 475 | 772 |

**Claims**

1. A water-absorbent resin comprising a polymer of a water-soluble ethylenically unsaturated monomer, which has a physiological saline absorption rate of 5 to 30 seconds, and has a diffusing absorption rate under load, which is expressed as a physiological saline absorption capacity under a load of 1.96 kPa in a local area of 78.5 mm$^2$, of 10 g/g or more.

2. The water-absorbent resin according to claim 1, which has a physiological saline retention capacity of 35 g/g or more.

3. The water-absorbent resin according to claim 1 or 2, which has a median particle size of 200 to 800 $\mu$m.

4. The water-absorbent resin according to any one of claims 1 to 3, which has at least one shape selected from the group consisting of a granular shape, a substantially spherical shape, and a shape in which particles having a granular shape and/or a substantially spherical shape are aggregated.

5. The water-absorbent resin according to any one of claims 1 to 4, which is one obtained by a reversed phase suspension polymerization method.

6. An absorbent article comprising an absorbent body including the water-absorbent resin according to any one of claims 1 to 5 and a hydrophilic fiber.

## INTERNATIONAL SEARCH REPORT

International application No.

PCT/JP2019/001322

### A. CLASSIFICATION OF SUBJECT MATTER

Int.Cl. C08F20/06(2006.01)i, B01J20/26(2006.01)i, B01J20/28(2006.01)i, B01J20/30(2006.01)i

According to International Patent Classification (IPC) or to both national classification and IPC

### B. FIELDS SEARCHED

Minimum documentation searched (classification system followed by classification symbols)

Int.Cl. C08F20/06, B01J20/26, B01J20/28, B01J20/30

Documentation searched other than minimum documentation to the extent that such documents are included in the fields searched

| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

CAplus/REGISTRY(STN)

### C. DOCUMENTS CONSIDERED TO BE RELEVANT

| Category* | Citation of document, with indication, where appropriate, of the relevant passages | Relevant to claim No. |
|---|---|---|
| X | WO 2013/031654 A1 (SUMITOMO SEIKA CHEMICALS CO., LTD.) 07 March 2013, claims, paragraph [0111], examples & US 2014/0194574 A1, claims, paragraph [0126], examples & EP 2752430 A1 & CN 103764685 A | 1–6 |
| A | JP 2012-41419 A (SUMITOMO SEIKA CHEMICALS CO., LTD.) 01 March 2012, claims, examples (Family: none) | 1–6 |

☒ Further documents are listed in the continuation of Box C. ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 31.01.2019 | 12.02.2019 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

International application No.

PCT/JP2019/001322

C (Continuation). DOCUMENTS CONSIDERED TO BE RELEVANT

| Category* | Citation of document, with indication, where appropriate, of the relevant passages | Relevant to claim No. |
|---|---|---|
| A | WO 2015/016075 A1 (SUMITOMO SEIKA CHEMICALS CO., LTD.) 05 February 2015, claims, examples & EP 3029077 A1, claims, examples & CN 105408366 A & TW 201509959 A | 1-6 |
| A | WO 2013/051417 A1 (SUMITOMO SEIKA CHEMICALS CO., LTD.) 11 April 2013, claims, examples & US 2014/0243478 A1, claims, examples & CN 103857705 A | 1-6 |
| A | JP 2000-143720 A (SANYO CHEMICAL IND LTD.) 26 May 2000, claims, examples (Family: none) | 1-6 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006176570 A
- WO 2013018571 A